# EUROPEAN PATENT SPECIFICATION

(11) **EP 0 973 490 B1**
(45) Date of publication and mention of the grant of the patent: **26.02.2003**
(21) Application number: 98913942.3
(22) Date of filing: 30.03.1998
(51) Int. Cl.: A61K 7/155

(54) **DEPILATORY COMPOSITIONS, THEIR PREPARATION AND USE**
ENTHAARUNGSZUSAMMENSETZUNGEN, DIE BEREITUNG UND DIE VERWENDUNG DERSELBEN
COMPOSITIONS DEPILATOIRES, LEUR PREPARATION ET LEUR UTILISATION

(30) Priority: 09.04.1997 EP 97400811; 04.06.1997 GB 9711447
(43) Date of publication of application: 26.01.2000
(73) Proprietor: Reckitt Benckiser France, 91301 Massy Cédex (FR)
(72) Inventor: GUILLAUME, Bruno, Craches F-78660 Prunay en Yvelines (FR); LEDON, Philippe, F-28300 Saint Prest (FR); CANELAS, Annick, F-28630 Le Coudray (FR); ACHER, David, F-28000 Chartres (FR); HEMERY, Séverine, F-27320 Nonancourt (FR); DAHMS, Gerd, D-42549 Velbert (DE); DESMOTS, Sarah, F-28100 Dreux (FR); DELAGNEAU, Hubert, F-28300 Mainvilliers (FR)
(74) Representative: Dickson, Elizabeth Anne
(86) International application number: GB9800950
(87) International publication number: WO98044898

(56) References cited:
- EP-A- 0 196 896
- WO-A-91/02538
- FR-A- 2 751 873
- US-A- 4 842 610
- DATABASE "CHEMICAL ABSTRACTS" (HOST: STN), Abs.110: 198 956, Colombus, OH, USA; & JP 63 313 618 (OSAKA AEROSOL IND. CO.) 21 December 1988 XP002071920

## Description

This invention relates to depilatory compositions, in particular those in the form of a gel.

Compositions for removing superfluous body hair are well known. These are of various types. One type of composition is in the form of a cream containing a depilatory agent which degrades hair keratin. In the case of a depilatory cream, hair is removed by applying the cream to an area of skin to be depilated, leaving the cream on the skin for sufficient time for the hair to be chemically dissolved, and then wiping and/or washing the cream and unwanted hair from the skin. Another type of composition is in the form of a wax, which is initially heated before being applied to the skin in a generally molten state. It is then allowed to solidify before being removed from the skin together with unwanted hair.

British patent specification no. GB 2 223 170 discloses a two-part depilatory composition in which the first part comprises polyvinyl alcohol (PVA) and the second part comprises borax and a polyhydroxy alcohol, or Congo red; the second part functioning to effect a controlled rate of cross-linking of the PVA. After in situ cross-linking, such compositions solidify, requiring the use of a plasticiser to enable the solid to be peeled from the skin. Such compositions preferably also include a surfactant.

Depilatory compositions in the form of a gel are less common and attempts to market such gels have had only limited success. The gels tended to have a higher skin irritancy than cream depilatories and became very sticky when being rinsed off because of the nature of the polymer present in the formulation. Moreover, depilatory compositions need to be of high pH, and problems were encountered in formulating a gel which would be stable in such highly alkaline conditions.

It is an aim of the present invention to provide a depilatory composition in the form of a gel which does not suffer from these disadvantages.

It is a further aim of the present invention to provide a depilatory composition in the form of a gel which may be sprayed onto the skin without propellant.

It is another aim of the present invention to provide a depilatory gel having a reduced tendency to irritate the skin.

It has been found that these aims can be achieved by forming a gel from two water-soluble polymeric components and in a pH environment maintained by a preferred buffer system. One of the polymeric components provides the gel with appropriate viscosity and the other polymeric component contributes to the stability of the gel.

Accordingly, one aspect of the present invention provides a depilatory composition in the form of an aqueous alkaline gel comprising:-
(i) a first polymeric binder comprising a charged, cross-linked, water-soluble polymer;
(ii) a second polymeric binder comprising a linear, water-soluble polymer;
(iii) a depilatory agent; and
(iv) a buffer system capable of maintaining the pH of the composition at a value of from 10.5 to 13.0.

Preferably, the composition remains in the form of an aqueous alkaline gel in use.

According to another aspect of the present invention, there is provided a process for producing a depilatory composition, which process comprises:-
(i) forming a first premix by mixing water and a first polymeric binder comprising a charged, cross-linked, water-soluble polymer; forming a second premix by mixing water, a buffer and a second polymeric binder comprising a linear, water-soluble polymer; and optionally forming a third premix by mixing a depilatory agent and a keratin degradation accelerator;
(ii) adding a depilatory agent or the third premix to the second premix with stirring;
(iii)adding the first premix to the resultant mixture with stirring; and
(iv) adjusting the pH to between 10.5 and 13.0.

The depilatory compositions of the present invention accordingly possess unique rheological qualities, which can be defined *inter alia* by their characteristic viscosity properties. For example, the shear rate (in seconds⁻¹) needed to decrease the viscosity of the gel by half is in the range of from about 10⁻² s⁻¹ to about 200 s⁻¹, preferably from about 1 s⁻¹ to about 50 s⁻¹. Such a large drop in the viscosity of depilatory compositions is not seen with prior art formulations over these shear rate ranges. At a low shear rate of about 10⁻² s⁻¹, the viscosity of the compositions of the present invention is of the order of 10³ Pas (10⁶ cP), whereas at a high shear rate of about 500 s⁻¹, their viscosity is of the order of about 0.1 Pas (100 cP). At a very high shear rate of the order of 4x10⁴ s⁻¹, such as that effected by a spray-pump (see Table 2 below), it is extremely difficult to measure viscosity, but it can be envisaged that that of the compositions of the present invention would be very low.

Preferably, a gel in accordance with the present invention has unique viscosity and elasticity, sustained high ionic strength and high pH.

The cross-linked polymer of the first component is such that it swells at high pH (pH 10.5 to 13) and preferably has only carbon-carbon bond cross-linkages. More preferably, the elastic modulus of the first component is in the range of from 200 Pa to 1000 Pa, conveniently, 400 Pa to 600 Pa, when measured at a frequency of 10 rad/s; at a temperature of 20°C and a strain of 0.1. The elastic (storage) modulus (G') corresponds to the energy which can be stored and released by the material.

Suitable first components include a cross-linked acrylic polymer such as a carbomer, for example, Ultrez 10 and/or Carbopol ETD 2020 from Goodrich, and Structure 2000 and 3001 from National Starch. Another suitable polymer is a cross-linked polyacrylamide such as Seppigel 305. Most preferably, the polymer of the first component is a copolymer of methyl vinyl ether and maleic anhydride cross-linked with dodecadiene available from ISP under the trade designation Antaron Stabilise 06.

Preferably, the depilatory composition includes from 0.1% to 3.0% by weight of the first component.

The second component is a linear non-ionic polymer and/or a positively charged polymer and/or a negatively charged polymer. The second, linear component is one which acts as a suspending agent for the gel and moderates the elastic modulus thereof to enable spreadability. Any suitable linear polymer may be chosen which enables the gel structure to be maintained at the osmotic pressure in the composition. Such linear polymers include hydrophilic polymers having a molecular weight of less than about 600 and non-ionic gel-forming polymers. For example, the second component may comprise a water-soluble non-ionic polymer such as polyethylenimine, polyethylene oxide or polyethylene glycol (PEG), a hydrophobically-modified polyol such as Acrysol 44 from Rohm and Haas, or a polyvinyl pyrrolidone such as PVP K from ISP or Luviskol from BASF.

Alternatively, or additionally, the second component may comprise a natural water-soluble polymer such as cellulose, hydroxypropyl cellulose, cetyl hydroxyethyl or propyl cellulose (for example Natrosol Plus from Aqualon), starch, polyglucoside, a polysaccharide such as an alginate, guar gum, carrageenan (lambda, kappa, iota), a sclerotium gum, a polypeptide with or without any alkyl chain (C1 to C18) modification and cationic end groups such as quaternary ammonium groups with counter ions such as chlorides and bromides, or a copolymer of sodium acrylate and dimethyl diallyl ammonium chloride (such as that known as Merquat), or a copolymer of hydroxy ethyl cellulose and dimethyl diallyl ammonium chloride (such as that known as Celquat).

Most preferably, the second component comprises polyethylenimine (such as that known under the trade name Lupasol from BASF) and/or polyvinyl pyrrolidone (such as that known under the trade designation PVP K-30 from ISP).

Preferably, the depilatory composition includes from 0.2% to 3.0% by weight of the second component.

The first and second components, when combined in accordance with the present invention, form a polymeric binder having unique viscosity and elasticity.

The depilatory agent may comprise any substance capable of degrading keratin and may be, for example, a sulphur compound such as potassium thioglycolate, dipotassium thioglycolate or thioglycerol.

Preferably, the depilatory composition includes from 0.1% to 6.0% by weight of depilatory agent, preferably from about 3.0% to 5.0%.

Also, the composition may optionally include component(s) which will accelerate the keratin reduction reaction such as urea, thiourea or dithioerythritol, dimethyl isosorbide (DMI), ethoxydiglycol (Transcutol) and methyl propyldiol (MP diol). DMI is preferred.

It is important that the pH of the composition is in the range of from about 10.5 to 13, and preferably about 11.0 to 12.5, to provide good depilation within about 5 minutes. In order to achieve this, a suitable buffer should be present, such as sodium silicate and/or L-arginine, sodium nicotinate and potassium polyethylenimine or a phosphate.

Generally, the buffer system will be present in an amount of from about 2.0% to 8.0% by weight, preferably from about 2.0% to 4.0% by weight.

Advantageously, the buffer system comprises from 2.0% to 3.0% by weight of L-arginine and from 0 to 1.0% by weight of polyethylenimine.

The pH buffer system preferably ensures efficacy of the depilation within 5 minutes whilst also minimizing skin irritation.

In order to achieve minimum skin irritation, a composition including L-arginine and/or polyethylenimine, urea and potassium and/or dipotassium thioglycolate is preferred.

In accordance with a preferred depilatory composition of the present invention, there is provided an aqueous alkaline gel comprising:
(i) 0.1% to 3.0% w/w of a first polymeric binder comprising a charged, cross-linked, water-soluble polymer, preferably a copolymer of methyl vinyl ether and maleic anhydride cross-linked with dodecadiene;
(ii) 0.2% to 3.0% w/w of a second polymeric binder comprising a linear, water-soluble polymer, preferably polyethylenimine and/or polyvinyl pyrrolidone;
(iii)0.1% to 6.0% w/w, preferably 3.0% to 5.0% w/w of a depilatory agent, preferably potassium and/or dipotassium thioglycolate;
(iv) 2.0% to 6.0% w/w, preferably 2.0% to 4.0% w/w of a buffer system, preferably L-arginine and/or polyethylenimine; and, optionally,
(v) 5.0% to 10.0% w/w of an acelerator for the degradation of keratin, preferably urea;
whereby the composition preferably remains in the form of an aqueous alkaline gel in use.

The gels according to the present invention are preferably transparent and remain transparent in use, depending on the amount of active ingredients present and on the procedure of mixing. Generally, they have a thick, rich feel and excellent rinsability with water. Moreover it is not necessary to include a surfactant in the compositions of the present invention, and hence they are less likely to cause skin damage.

Due to the special rheology of the polymeric binder, it is possible to include beads, dyes and/or oily particles which will remain dispersed in the gel. Optional excipients include compounds for soothing the skin such as azulene or jojoba and polyethylene glycol ester to reduce irritation. Suitable beads include encapsulated algae extract and sweet almond oil (Elespher). It is also possible to include hydrating, accelerating and/or reducing agents in beads. For example, water-soluble materials such as urea, potassium thioglycolate or thioglycerol can be included in beads, so that these actives are encapsulated.

Although it is possible to provide a gel in accordance with the present invention that has visco-elastic properties suitable for spraying, the depilatory material may alternatively be applied by means of a roll-on device or in conventional shower gel packaging.

Other ingredients may be included in the compositions of the invention, such as are known in the art.

Unless otherwise stated, all percentage weights referred to herein are % weights based on the weight of the total composition.

For a better understanding of the invention and to show how the same may be carried into effect, reference will now be made, by way of example, to the accompanying drawings, in which:-
Figure 1 is a graph of shear stress (Pa) against shear rate (s⁻¹) for a series of polymeric binders (first component) for the composition of the invention;
Figure 2 is a graph of the yield points (Pa) of the binders of Figure 1 against the cross-linked polymer (first component) contents (% w/w) of the binders;
Figure 3 is a graph of viscosities (Pas) of the binders of Figure 1 against the shear rate (s⁻¹) of the binders; and

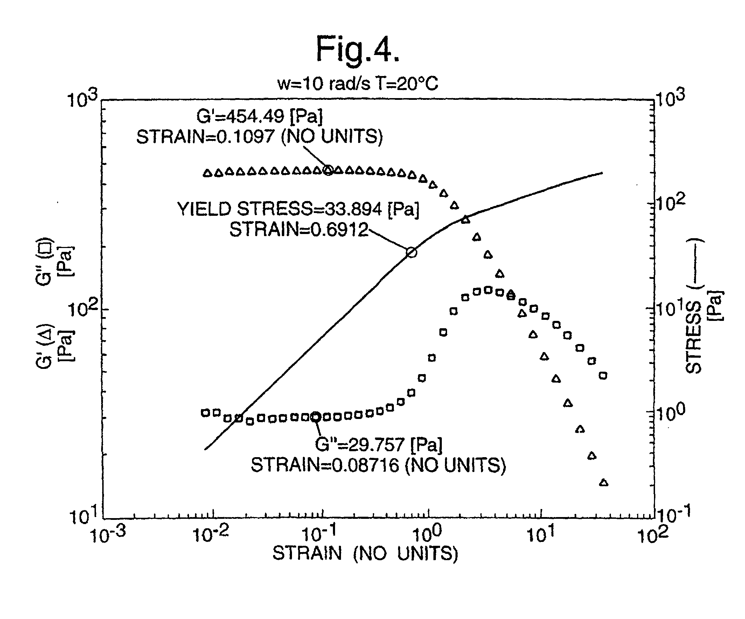

Figure 4 is a graph of the elastic (G') and viscous (G'') moduli against stress/strain (Pa) of the first component of the composition.

The following Examples illustrate the invention:

### EXAMPLES 1 TO 6

A series of depilatory gels was prepared having the compositions shown in the following Table 1:

**TABLE 1**

| | 1 | 2 | 3 | 4 | 5 | 6 |
|---|---|---|---|---|---|---|
| **Phase 1** | | | | | | |
| Polymer A | 3.0% | 2.5% | 2.25% | 2.0% | 1.5% | 1.0% |
| deionised water | 47.0% | 39.0% | 36.35% | 31.4% | 23.5% | 15.7% |

| **Phase 2** | | | | | | |
|---|---|---|---|---|---|---|
| Polymer B | 2.0% | 2.0% | 2.0% | 2.0% | 2.0% | 2.0% |
| pH buffer | 3.0% | 3.0% | 3.0% | 3.0% | 3.0% | 3.0% |
| deionised water | 10.7% | 19.2% | 24.8% | 26.4% | 38.3% | 45.8% |

| **Phase 3** | | | | | | |
|---|---|---|---|---|---|---|
| potassium thioglycolate (30%) | 14.7% | 14.7% | 14.7% | 14.7% | 14.7% | 14.7% |
| urea | 8.0% | 8.0% | 8.0% | 8.0% | 8.0% | 8.0% |

| **Final mix** | | | | | | |
|---|---|---|---|---|---|---|
| Phase 1 | 50.0% | 41.5% | 37.5% | 33.4% | 25.0% | 16.7% |
| Phase 2 | 15.7% | 24.2% | 29.8% | 33.4% | 43.3% | 50.8% |
| Phase 3 | 22.7% | 22.7% | 22.7% | 22.7% | 22.7% | 22.7% |
| Potassium hydroxide 50% | 11.6% | 11.6% | 10.0% | 10.5% | 9.0% | 9.8% |
| **Total** | 100% | 100% | 100% | 100% | 100% | 100% |
| Total deionised water | 57.5% | 58.0% | 59.9% | 59.6% | 1.6% | 61.3% |

All amounts are expressed as % weight/weight total composition. Polymer A was a methyl vinyl ether/maleic anhydride copolymer cross-linked with dodecadiene known under the trade designation Antaron ST06 from ISP; and polymer B was polyvinyl pyrrolidone known under the trade designation PVP K-30 from ISP. The pH buffer system was a combination of 2% w/w of L-arginine and 1% w/w of polyethylenimine.

In each case, an Olsa mixer with planar stirring and a high shear stirrer equipped with a vacuum and heating/cooling system was used. The procedure was as follows:

### Phase 1: Premix polymer A and water.

The water was placed in the mixer and polymer A was added slowly with stirring. When the polymer had been dispersed, heating was commenced whilst maintaining a vacuum of about 0.5 to 0.8 bar to avoid air bubbles. The vacuum pump was operating intermittently in order to reduce water loss. When the temperature had reached about 80°C an almost clear gel had been formed. This was allowed to cool and swell overnight.

### Phase 2: Premix polymer B, water, pH buffer

The water and polymer B were introduced into the mixer and homogenised for 2 minutes. The pH buffer system was then added and the mixture homogenised for a further 2 minutes.

### Phase 3: Urea and potassium thioglycolate

The urea was added to the potassium thioglycolate in a beaker and stirred for 1 hour to complete dissolution.

**Final mixing:** The final mixing was effected as follows:-

Phase 2 was placed in the vessel and phase 3 was added with stirring for 15 minutes. A white precipitate appeared. Phase 1 was added while mixing slowly under vacuum (0.5 bar) with planar stirring and the high shear stirrer operated at low speed. The mixture was homogenised for 30 minutes until a lumpy gel had been formed. The potassium hydroxide was then added. The gel became clear and transparent. The pH was adjusted, as necessary, to from 11.0 to 12.5.

Each of the resultant gels was subjected to rheological measurement. The parameters considered were shear stress, shear rate, viscosity and yield point. The results are shown in the Figures.

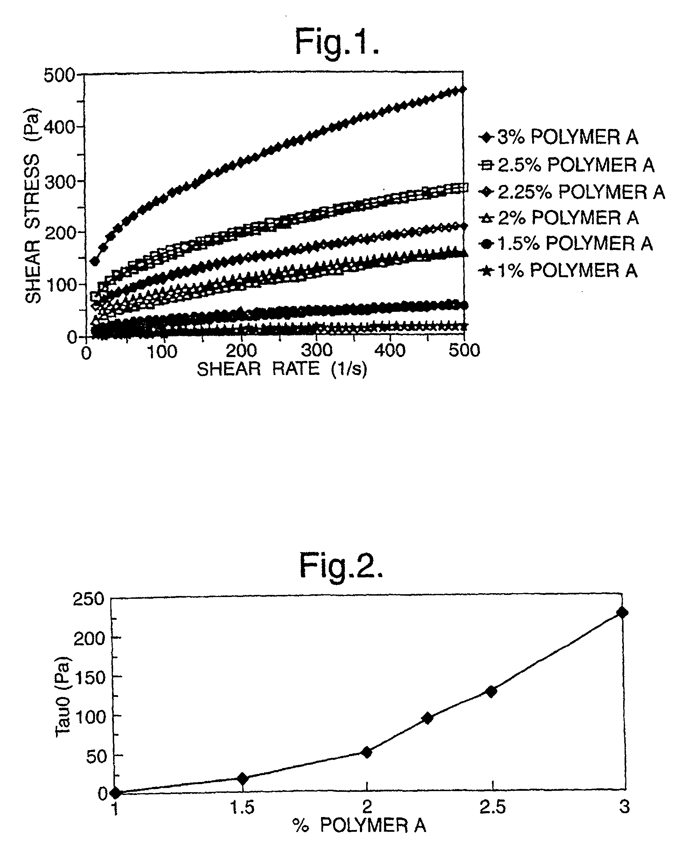

As can be seen from Figure 1, the variation of shear stress with shear rate is non-linear between 0 and 500 s⁻¹ and thus the gel has a non-Newtonian behaviour with a yield point. The yield point is the stress required to make the gel flow. Figure 2 shows that the variation of the yield point (TauO), i.e. the shear stress for zero shear rate, varies exponentially. For a small variation in the amount of polymer A, the variation in the yield point, i.e. the variation in spreadability or consistency, may be significant.

The following table, Table 2, gives characteristic shear rates of products to be sprayed with a conventional pump spray and products to be applied from a shower gel packaging.

**TABLE 2**

| Type of packaging | Flow Rate (from packaging) of product (cm³/s) | Radius of the external output (cm) | Shear rate (s⁻¹) |
|---|---|---|---|
| Pump Spray | 0.17 | 0.0175 (radius of the actuator) | 500-40000 |
| Shower gel packaging | 8.8 | ca 0.15 | 500-30000 |

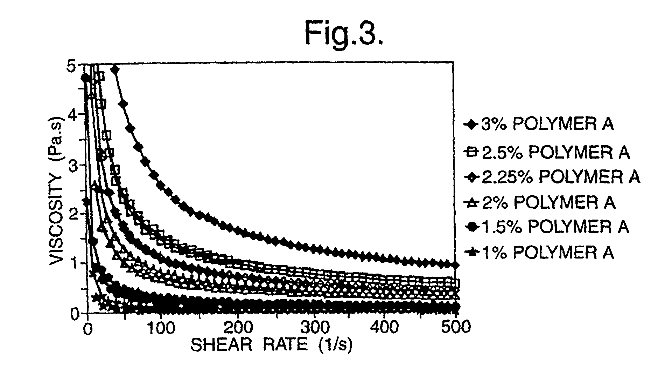

The relationship between shear rate and viscosity is shown in Figure 3, from which it can be seen that, at the shear rates given in Table 2, the viscosity is very low.

It has been found that, in order for the gel to be sprayable with a pump spray, its yield point should be not more than from 100 to 200 Pa. If, however, the gel is to be used in a shower gel pack, the yield point may be as high as 300 Pa. Also, in order for the product to remain still for 5 minutes on the skin when held vertical, the yield point needs to be about 60 Pa or more. Thus gels wherein polymer A constitutes from 2.0% to 2.5% w/w are preferred for spraying, whereas rather lower percentages of polymer A may be present where the gel is to be used in the form of a shower gel packaging.

In a further series of experiments, the blue powder Azulene was dissolved in thick paraffin oil to produce a 1% w/w solution. 2% w/w of this solution was then slowly added to each of the above gels and stirred for 5 minutes. The Azulene remained stably suspended within the gel in the form of blue oily droplets. Depending upon the concentration of Azulene in the premix and the concentration of premix in the gel, the size and colour of the droplets and the overall transparency of the gel could be adjusted.

In a further series of experiments, 0.5% w/w of Elespher was slowly added to each gel and stirred for 5 minutes. Elespher consists of encapsulated beads of algae extract and sweet almond oil, and is green in colour. The beads were stably suspended in the gel.

### EXAMPLE 7

Gels were made in accordance with the following Table 3:

**TABLE 3**

| | **%w/w** |
|---|---|
| **Phase 1** | |
| polymer A | 1-3 |
| water | 40.0 |
| KOH 50% | 4.0 |
| Total phase 1 | 47.0 |

| **Phase 2** | |
|---|---|
| polymer B | 0.6-3 |
| buffer system | 3.0 |
| water | 8.2 |
| Total phase 2 | 11.8 |

| **Phase 3** | |
|---|---|
| water | 6.5 |
| potassium thioglycolate (30%) | 14.7 |
| Total phase 3 | 29.2 |
| water | to 100.0 |
| KOH 50% | 6.15 |

The amount of water was varied depending upon the amounts of polymers A and B present. Polymers A and B were as used in Examples 1 to 6 and the mixing technique employed was as described in those Examples. In each case, stable, sprayable depilatory gels were obtained. This showed that for a range of polymer A between 1.0% and 3.0%, a range of polymer B between 0.6% and 3.0% could be used and a stable, sprayable gel formed.

## Claims

1. A depilatory composition in the form of an aqueous alkaline gel comprising:
(i) a first polymeric binder comprising a charged, cross-linked, water-soluble polymer;
(ii) a second polymeric binder comprising a linear, water-soluble polymer;
(iii)a depilatory agent; and
(iv) a buffer system capable of maintaining the pH of the composition at a value of from 10.5 to 13.0.

2. A depilatory composition as claimed in claim 1, wherein the cross-linked polymer of the first component is a cross-linked acrylic polymer, a cross-linked polyacrylamide or a copolymer of methyl vinyl ether and maleic anhydride cross-linked with dodecadiene.

3. A depilatory composition as claimed in claim 1 or 2, which includes from 0.1 to 3.0% w/w of the first component.

4. A depilatory composition as claimed in any of claims 1 to 3, wherein the second component comprises a linear, non-ionic polymer and/or positively charged polymer and/or negatively charged polymer.

5. A depilatory composition as claimed in claim 4, wherein the second component comprises one or more of polyethylenimine, polyvinyl pyrrolidone, polyethylene oxide, polyethylene glycol, cellulose, hydroxy propyl cellulose, cetyl hydroxy ethyl cellulose, cetyl hydroxy propyl cellulose, starch, a polyglucoside, a polysaccharide, guar gum, carrageenan, a sclerotium gum, a polypeptide, and a copolymer of sodium acrylate or of hydroxy ethyl cellulose with dimethyl diallyl ammonium chloride.

6. A depilatory composition as claimed in any preceding claim, which includes from 0.2 to 3.0% w/w of the second component.

7. A depilatory composition as claimed in any preceding claim, wherein the depilatory agent is potassium thioglycolate or thioglycerol.

8. A depilatory composition as claimed in any preceding claim, including an accelerator for the degradation of keratin, such as urea, thiourea, dithioerythritol, ethoxydiglycol or methylpropyldiol.

9. A depilatory composition as claimed in any preceding claim, including a buffer system comprising a silicate, arginine such as L-arginine, a nicotinate, polyethylenimine or a phosphate.

10. A depilatory composition as claimed in any preceding claim and having a yield point of not more than 200 Pa.

11. A depilatory composition as claimed in any preceding claim, wherein the shear rate needed to decrease the viscosity of the gel by half is in the range of from 10⁻² s⁻¹ to 200 s⁻¹, preferably from 1 s⁻¹ to 50 s⁻¹.

12. A depilatory composition as claimed in any preceding claim, in the form of a sprayable gel.

13. A process for producing a depilatory composition as claimed in any preceding claim, which process comprises:-
(i) forming a first premix by mixing water and the first component; forming a second premix by mixing water, a buffer and the second component; and optionally forming a third premix by mixing a depilatory agent and a keratin degradation accelerator;
(ii) adding a depilatory agent or the third premix to the second premix with stirring;
(iii)adding the first premix to the resultant mixture with stirring; and
(iv) adjusting the pH to between 10.5 to 13.0.

## Patentansprüche

1. Enthaarungszusammensetzung in der Form eines wässrigen, alkalischen Gels, umfassend:
(i) ein erstes polymeres Bindemittel, umfassend ein geladenes, vernetztes, wasserlösliches Polymer;
(ii) ein zweites polymeres Bindemittel, umfassend ein lineares, wasserlösliches Polymer;
(iii) ein Enthaarungsmittel; und
(iv) ein Puffersystem, geeignet den pH-Wert der Zusammensetzung auf einem Wert von 10,5 bis 13,0 zu halten.

2. Enthaarungszusammensetzung gemäß Anspruch 1, wobei das vernetzte Polymer der ersten Komponente ein vernetztes Acrylpolymer, ein vernetztes Polyacrylamid oder ein mit Dodecadien vernetztes Copolymer aus Methylvinylether und Maleinsäureanhydrid ist.

3. Enthaarungszusammensetzung gemäß Anspruch 1 oder 2, welches 0,1 bis 3,0 % w/w der ersten Komponente enthält.

4. Enthaarungszusammensetzung gemäß einem der Ansprüche 1 bis 3, wobei die zweite Komponente ein lineares, nichtionisches Polymer und/oder ein positiv geladenes Polymer und/oder ein negativ geladenes Polymer umfasst.

5. Enthaarungszusammensetzung gemäß Anspruch 4, wobei die zweite Komponente einen oder mehrere Bestandteile aus Polyethylenimin, Polyvinylpyrrolidon, Polyethylenoxid, Polyethylenglycol, Cellulose, Hydroxypropylcellulose, Cetylhydroxyethylcellulose, Cetylhydroxypropylcellulose, Stärke, einem Polyglucosid, einem Polysaccharid, Guargummi, Carrageenan, einem Sclerotiumgummi, einem Polypeptid und einem Copolymer aus Natriumacrylat oder aus Hydroxyethylcellulose mit Dimethyldiallylammoniumchlorid umfasst.

6. Enthaarungszusammensetzung gemäß einem der vorstehenden Ansprüche, welches 0,2 bis 3,0 % w/w der zweiten Komponente enthält.

7. Enthaarungszusammensetzung gemäß einem der vorstehenden Ansprüche, wobei das Enthaarungsmittel Kaliumthioglycolat oder Thioglycerin ist.

8. Enthaarungszusammensetzung gemäß einem der vorstehenden Ansprüche, welches einen Beschleuniger für den Abbau von Keratin enthält, wie Harnstoff, Thioharnstoff, Dithioerythritol, Ethoxydiglycol oder Methylpropyldiol.

9. Enthaarungszusammensetzung gemäß einem der vorstehenden Ansprüche, welches ein Puffersystem, umfassend ein Silikat, Arginin, wie L-Arginin, ein Nikotinat, Polyethylenimin oder ein Phosphat, enthält.

10. Enthaarungszusammensetzung gemäß einem der vorstehenden Ansprüche mit einem Fließpunkt von nicht mehr als 200 Pa.

11. Enthaarungszusammensetzung gemäß einem der vorstehenden Ansprüche, wobei die Schergeschwindigkeit, die benötigt wird, um die Viskosität des Gels um die Hälfte zu verringern, im Bereich von 10⁻² s⁻¹ bis 200 s⁻¹, bevorzugt von 1 s⁻¹ bis 50 s⁻¹, liegt.

12. Enthaarungszusammensetzung gemäß einem der vorstehenden Ansprüche in der Form eines sprühbaren Gels.

13. Verfahren zur Herstellung einer Enthaarungszusammensetzung nach einem der vorstehenden Ansprüche, umfassend:
(i) Bilden eines ersten Vorgemisches durch Vermischen von Wasser und der ersten Komponente; Bilden eines zweiten Vorgemisches durch Vermischen von Wasser, einem Puffer und der zweiten Komponente; und gegebenenfalls Bilden eines dritten Vorgemisches durch Vermischen eines Enthaarungsmittels und eines Keratinabbaubeschleunigers;
(ii) Zugeben eines Enthaarungsmittels oder des dritten Vorgemisches zum zweiten Vorgemisch unter Rühren;
(iii) Zugeben des ersten Vorgemisches zum erhaltenen Gemisch unter Rühren; und
(iv) Einstellen des pH-Wertes auf einenWert zwischen 10,5 und 13,0.

## Revendications

1. Composition épilatoire sous forme d'un gel alcalin aqueux comprenant :
(i) un premier liant polymère comprenant un polymère hydrosoluble réticulé chargé ;
(ii) un second liant polymère comprenant un polymère hydrosoluble linéaire ;
(iii) un agent épilatoire ; et
(iv) un système tampon capable de maintenir le pH de la composition à une valeur dans la plage de 10,5 à 13,0.

2. Composition épilatoire suivant la revendication 1, dans laquelle le polymère réticulé du premier constituant est un polymère acrylique réticulé, un polyacrylamide réticulé ou un copolymère d'éther méthylique de vinyle et d'anhydride maléique réticulé avec du dodécadiène.

3. Composition épilatoire suivant la revendication 1 ou 2, qui comprend 0,1 à 3,0 % en poids/poids du premier constituant.

4. Composition épilatoire suivant l'une quelconque des revendications 1 à 3, dans laquelle le second constituant comprend un polymère linéaire non ionique et/ou un polymère chargé positivement et/ou un polymère chargé négativement.

5. Composition épilatoire suivant la revendication 4, dans laquelle le second constituant comprend un ou plusieurs des composés consistant en la polyéthylène-imine, la polyvinylpyrrolidone, le poly(oxyde d'éthylène), le polyéthylène-glycol, la cellulose, l'hydroxypropylcellulose, la cétylhydroxyéthylcellulose, la cétylhydroxypropylcellulose, l'amidon, un polyglycoside, un polysaccharide, la gomme guar, la carraghénine, une gomme de Sclerotium, un polypeptide et un copolymère d'acrylate de sodium et d'hydroxyéthylcellulose avec du chlorure de diméthyldiallylammonium.

6. Composition épilatoire suivant l'une quelconque des revendications précédentes, qui comprend 0,2 à 3,0 % en poids/poids du second constituant.

7. Composition épilatoire suivant l'une quelconque des revendications précédentes, dans laquelle l'agent épilatoire est le thioglycolate de potassium ou le thioglycérol.

8. Composition épilatoire suivant l'une quelconque des revendications précédentes, comprenant un accélérateur pour la dégradation de la kératine, tel que l'urée, la thiourée, le dithio-érythritol, l'éthoxydiglycol ou le méthylpropyldiol.

9. Composition épilatoire suivant l'une quelconque des revendications précédentes, comprenant un système tampon comprenant un silicate, de l'arginine telle que la L-arginine, un nicotinate, de la polyéthylène-imine ou un phosphate.

10. Composition épilatoire suivant l'une quelconque des revendications précédentes et ayant un point de fluage non supérieur à 200 Pa.

11. Composition épilatoire suivant l'une quelconque des revendications précédentes, dans laquelle le taux de cisaillement requis pour diminuer la viscosité du gel de moitié est compris dans l'intervalle de 10⁻² s⁻¹ à 200 s⁻¹, de préférence de 1 s⁻¹ à 50 s⁻¹.

12. Composition épilatoire suivant l'une quelconque des revendications précédentes, sous forme d'un gel atomisable.

13. Procédé pour la production d'une composition épilatoire suivant l'une quelconque des revendications précédentes, procédé qui comprend les étapes consistant :
(i) à former un premier prémélange en mélangeant de l'eau et le premier constituant ; à former un deuxième prémélange en mélangeant de l'eau, un tampon et le second constituant ; et, facultativement, à former un troisième prémélange en mélangeant un agent épilatoire et un accélérateur de dégradation de la kératine ;
(ii) à ajouter un agent épilatoire ou le troisième prémélange au deuxième prémélange, sous agitation ;
(iii) à ajouter le premier prémélange au mélange résultant, sous agitation ; et
(iv) à ajuster le pH dans la plage de 10,5 à 13,0.
